# EUROPEAN PATENT SPECIFICATION

(11) **EP 3 684 392 B1**
(45) Date of publication and mention of the grant of the patent: **19.08.2026**
(21) Application number: 18780249.1
(22) Date of filing: 21.09.2018
(51) Int. Cl.: A61K 36/63, A61K 31/05, A61P 9/00, A23L 2/00

(54) **EXTRACT OF OLIVE VEGETATION WATERS AND/OR OLIVE POMACE FOR USE IN TREATING DAMAGE TO THE CARDIOVASCULAR SYSTEM**
EIN EXTRAKT AUS OLIVENVEGETATIONSWASSER UND/ODER OLIVENTRESTER ZUR VERWENDUNG IN DER BEHANDLUNG VON SCHÄDEN DES KARDIOVASKULÄREN SYSTEMS
EXTRAIT D'EAU DE VÉGÉTATION D'OLIVE ET/OU MARC D'OLIVE POUR L'UTILISATION DANS LE TRAITEMENT D'UN DOMMAGE AU SYSTÈME CARDIOVASCULAIRE

(30) Priority: 22.09.2017 IT 201700106462
(43) Date of publication of application: 29.07.2020
(73) Proprietor: Fattoria La Vialla Di Gianni, Antonio E Bandino Lo Franco - Societa' Agricola Semplice, 52100 Arezzo (IT)
(72) Inventor: ALBINI, Adriana, 20099 Sesto San Giovanni (Milano) (IT); BRUNO, Antonino, 20156 Milano (IT); BACI, Denisa, 20873 Cavenago di Brianza (Monza-Brianza) (IT); MACRI', Nicoletta, 20068 Peschiera Borromeo (Milano) (IT); NOONAN, Douglas, 20099 Sesto San Giovanni (Milano) (IT)
(74) Representative: Biggi, Cristina
(86) International application number: PCT/IB2018/057307
(87) International publication number: WO 2019/058323

(56) References cited:
- EP-A1- 2 338 500
- WO-A2-2009/013596
- US-A1- 2014 155 339
- US-A1- 2014 296 141
- CLAUDE L. LÉGER: "Decreased Superoxide Anion Production in Cultured Human Promonocyte Cells (THP-1) Due to Polyphenol Mixtures from Olive Oil Processing Wastewaters", JOURNAL OF AGRICULTURAL AND FOOD CHEMISTRY, vol. 48, no. 10, 13 September 2000 (2000-09-13), US, pages 5061 - 5067, XP093166512, ISSN: 0021-8561, DOI: 10.1021/jf991349c

## Description

The present invention relates to a phytocomplex or natural concentrate, rich in polyphenol compounds, such as hydroxytyrosol and Oleuropeinaglycone di-aldehyde (3,4-DHPA-EDA), derived from the pressing waters of oil olives and/or from pomace from the olive grinding process, for use in the prevention and/or treatment of damage to the cardiovascular system, preferably in subjects affected by/suffering from hypertension, cardiac decompensation, arrhythmia, heart attacks or those subjected to chemotherapy.

### PRIOR ART

With the extension of the average life expectancy of the population, especially in developed countries, the risk of developing degenerative diseases affecting the cardiovascular system has therefore increased. Furthermore, such risk is often also increased by incorrect dietary habits and by an unhealthy lifestyle (stress, smoking, obesity, etc.). Subjects affected by hypertension, cardiac decompensation, arrhythmia, heart attacks or those subjected to chemotherapy are therefore the application target of the present invention.

While at one time the role of cardiovascular toxicity associated with antineoplastic therapies had a reduced impact on life expectancy with respect to the gain in survival guaranteed by the antineoplastic therapies themselves, there is currently an increase in the frequency of cardiovascular events in patients subjected to treatment with anti-cancer drugs or therapies; cardiovascular events therefore now represent an important risk factor in the course of cancer treatments.

Useful substances in cardiovascular protection particularly, but not exclusively, during the course of an antineoplastic treatment include beta blockers, ACE inhibitors, statins, anti-coagulants, whose use is now known and traditional, but recently compounds such as dexrazoxane, N-acetyl-L-cysteine (NAC), zinc, selenium, melatonin, L-carnitine, glutathione and coenzyme Q10 have started to gain ground, as per reference (Albini A. et al, JNCI 2009, PMID:20007921). Furthermore, substances of natural origin are being studied, in particular deriving from plants such as, for example, extracts of ginkgo biloba, red wine, grape, green tea, but also curry and chilli.

It is known that the incidence rate of cardiovascular diseases is substantially reduced in populations that adopt a Mediterranean diet based on the consumption of olive oil. This scientific evidence has provided an incentive for the study of olive oil in order to define the composition thereof and, in particular, in order to identify the substances with medical/pharmacological potential.

A characteristic of olive oil that has aroused particular interest has been the high polyphenol content contained therein. These compounds are natural antioxidants, of vegetable origin, able to inhibit the formation of free radicals.

The beneficial properties of olive oil have led to a substantial increase, especially in Italy, in the growing of olive trees and the production of oil, with a consequent increase in the production of collateral derivatives of olive oil, mainly vegetation waters and pomace, which are characterized by a high pollutant load and therefore generate a substantial environmental impact.

EP 2338500 discloses a concentrate comprising hydroxytyrosol obtained from residues form olive production with membrane filtration and reverse osmosis step and use for the treatment of hypertension.

WO2009/013596 relates to extracts form olive pomace or olive mill vegetation water comprising hydroxytyrosol for the treatment of cardiovascular disease.

US2014/155339 discloses compositions comprising hydroxytyrosol obtained form vegetation water of olive oil production for treating or preventing chemotherapy-induced dysfunction.

US 2014/296141 discloses compositions comprising hydroxytyrosol from olives for the treatment of cardiovascular disease or hypertension.

The disposal of this material is rigorously regulated both at national and regional level and the actuation of the legislation (Law 574 of 11/1996) implies large expense for growers who cannot gain any advantage from these waste products which are however rich in molecules with high medical/pharmaceutical potential.

Although a lot of research has been performed on vegetation waters, there is still a strongly felt need to identify new properties that can give value to these waste products which would otherwise exclusively represent a high cost for the grower and environmental contamination. There is a particularly felt need to identify new nutritional and/or medical/pharmaceutical properties to make use of this waste product.

On that point, the Applicant has surprisingly found that vegetation waters are able to perform a protective effect on the cardiovascular system (cardioprotection or cardioprevention effect), in particular the protection is performed on the cells of the heart muscle or myocardium - the cardiomyocytes, as demonstrated by experiments performed *in vivo* on zebrafish embryos (*Danio rerio*).

Specifically, the authors have demonstrated how, by concentrating the permeate of the vegetation water subjected to microfiltration through reverse osmosis, a phytocomplex is obtained that is rich in polyphenol compounds able to protect - *in vivo* - the cardiovascular system, in particular the cardiomyocytes.

This effect is particularly advantageous for human health as it guarantees effective cardioprotection action in particular, but not exclusively, in subjects exposed to cardiovascular risk such as subjects affected by hypertension, cardiac decompensation, arrhythmia, heart attacks or those subjected to chemotherapy. In particular, vegetation waters are able to exert a cardioprotection effect on the heart tissue, which may be damaged by treatment with antineoplastic agents, whose cardiovascular toxicity is known.

### BRIEF DESCRIPTION OF THE FIGURES

Further advantages of the present invention will be highlighted in the following detailed description and appended figures, in particular:

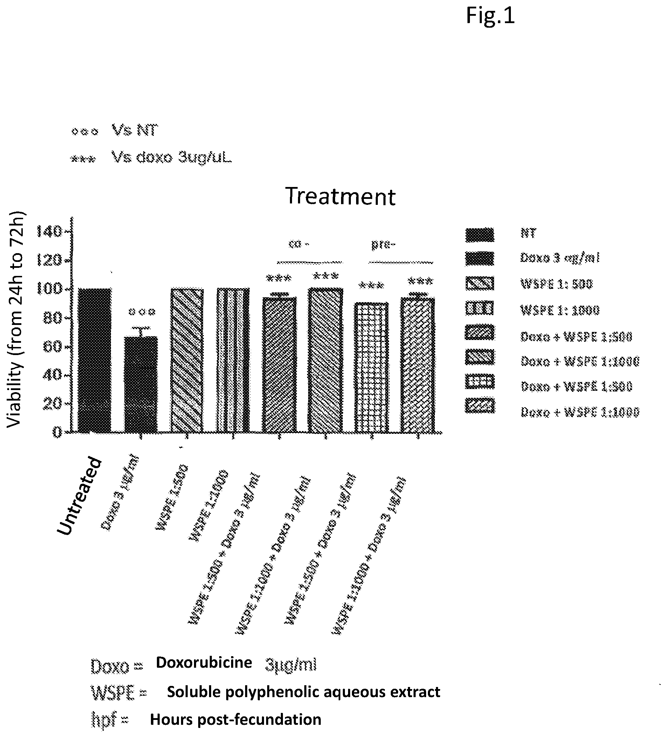

- Figure 1 shows the effects of the combined treatment of A009+Doxorubicin on the viability of zebrafish embryos, 72 hours post-fertilization (hpf). The embryos were treated with the polyphenol concentrate of the present invention (sample A009), at the dilutions 1:100 and 1:500, alone or in association with Doxorubicin (3 µg/mL). A pre-treatment was performed with A009, followed by administration of Doxorubicin and A009+Doxorubicin co-treatment. NT = not-treated cells. °°° = p<0.0001 with respect to not-treated embryos; *** = p<0.001 with respect to embryos treated with doxorubicin alone;

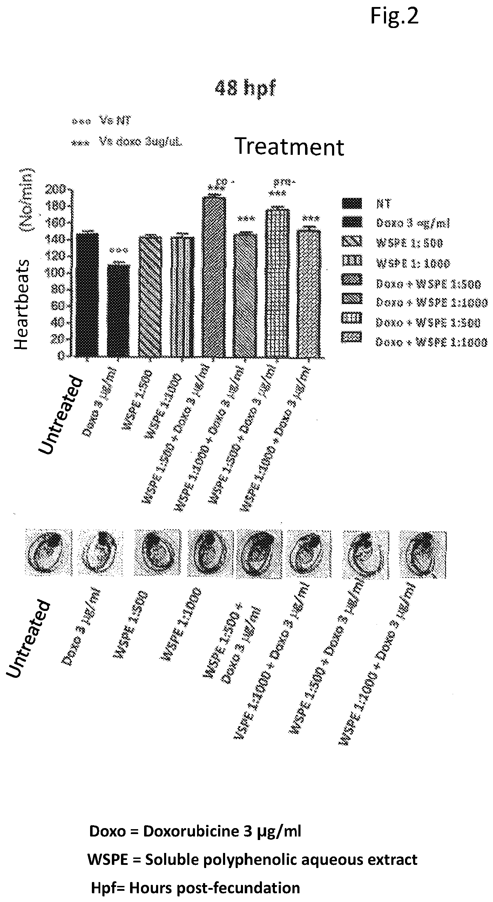

- Figure 2 shows the effects of the combined treatment (48 hours) of A009+Doxorubicin on the heart rate of zebrafish embryos. The embryos were treated with the polyphenol concentrate of the present invention (sample A009), at the dilutions 1:100 and 1:500, alone or in association with Doxorubicin (3 µg/mL). A pre-treatment was performed with A009, followed by administration of Doxorubicin and A009+Doxorubicin co-treatment. NT = not-treated cells. °°° = p<0.0001 with respect to not-treated embryos; *** = p<0.001 with respect to embryos treated with doxorubicin alone;

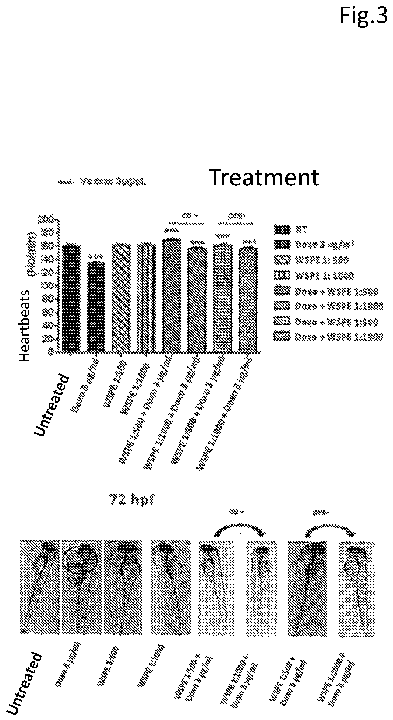

- Figure 3 shows the effects of the combined treatment (72 hours) of A009+Doxorubicin on the heart rate of zebrafish embryos. The embryos were treated with the polyphenol concentrate of the present invention (sample A009), at the dilutions 1:100 and 1:500, alone or in association with Doxorubicin (3 µg/mL). A pre-treatment was performed with A009, followed by administration of Doxorubicin and A009+Doxorubicin co-treatment. NT = not-treated cells. °°° = p<0.0001 with respect to not-treated embryos; *** = p<0.001 with respect to embryos treated with doxorubicin alone.

### DETAILED DESCRIPTION OF THE INVENTION

A first aspect of the invention regards a phytocomplex or concentrate of olive vegetation waters and/or pomace comprising hydroxytyrosol and/or 3,4-DHPA-EDA, for use in the prevention and/or treatment of damage caused by drug and/or chemotherapy agent having a cardiotoxic effect. The concentrate is obtained by a process comprising the steps of:
(i) microfiltering a sample of the vegetation water and/or olive pomace so as to obtain a concentrate and a permeate of microfiltration; and
(ii) concentrating by reverse osmosis the microfiltration permeate of step (i),and the quantity of 3,4-DHPA-EDA is comprised between 0.5 and 8 g/L, preferably between 1 and 6 g/L , more preferably between 1.5 and 2.5 g/L. t. In other words, the phytocomplex or concentrate of vegetation waters and/or pomace of the present invention is used for preventive and/or protective purposes, in particular for cardioprotection or cardioprevention, or for therapeutic purposes for treating damage to the cardiovascular system caused by/associated with the pharmacological treatment, preferably chemotherapy and/or radiotherapy and/or any pharmacological treatment associated with side effects that involve the cardiovascular compartment.

Any drug and/or chemotherapy agent having a cardiotoxic effect is included in the present invention, preferably said drug and/or chemotherapy agent such as, for example, anthracycline, capecitabine, citabine, 5-fluorouracil, third-generation aromatase inhibitors, cyclophosphamide, Imatinib, Sorafenib, Sunitinib, SERMs, Trastuzumab, Bevacizumab, specific COX-2 inhibitors (Albini A. et al, JNCI 2009, PMID:20007921).

Reference will be made below to this phytocomplex or concentrate simply with the term "concentrate" or "polyphenol concentrate". In fact, as will be demonstrated in more detail in the example of the present description on the zebrafish animal model, the concentrate of vegetation waters and/or pomace has demonstrated therapeutically significant efficacy in the reduction and/or prevention of damage to the cardiovascular system, in particular heart damage, both in terms of reducing mortality and normalizing the heart rate. In particular, in the example the induction of cardiotoxic damage was used through doxorubicin chemotherapy that imitates the cardiac damage induced by drugs able to induce phenotypic, biochemical and functional alterations to the heart muscle.

The cardiovascular system or apparatus is comprised of a muscular pump (the heart) and a series of conduits (the blood vessels) in which a fluid (blood) moves, which is pumped all over the body. The heart and vessels are indicated overall as the circulatory/cardiocirculatory system or apparatus. The task of the blood that moves in the circulatory apparatus is to transport and transfer respiratory gases, nutrients, waste products, hormones, cells and molecules of the immune system and heat from one tissue to another. All these functions are essential in order to guarantee a stable internal environment and communication between cells.

In the context of the present invention, damage to the cardiovascular system as defined above also means a lesion, a reaction or a side effect. In the context of the present invention, cardiovascular damage as defined above means any phenotypic and/or anatomical and/or functional alteration to the cardiovascular system, preferably to the heart muscle (myocardium) and/or blood vessels (vascular system). Preferably, said damage is caused/provoked by or associated with one or more reasons, preferably selected from: pathological conditions, advanced age, harmful lifestyles and taking medicines. Preferably, said damage is caused/provoked by chemotherapy and/or radiotherapy or is a damage due to cardiotoxicity. Preferably, said damage is selected from: hypertensive crisis, pulse alterations, arrhythmia, ischemia, preferably caused by vasospasm and/or by thromboembolic phenomena, fibrosis, loss of contractile activity for the cardiac counterpart, cell senescence, production of reactive oxygen species and autophagy, both for the cardiac component and the vascular one.

According to a preferred aspect of the invention, said damage affects the cardiomyocytes and/or the endothelial cells of the cardiovascular system, preferably those of the blood vessels.

According to a preferred aspect of the invention, said concentrate is used during and/or following, and/or prior to radiotherapy treatment and/or treatment with drugs, preferably drugs having a cardiotoxic action, preferably antineoplastic drugs, for which there is a known correlation with the onset of vascular and/or cardiac lesions. In other words, the concentrate of the invention can perform a cardioprotection action, i.e. protection against the adverse effects associated with/caused by radiotherapy and/or drugs, preferably chemotherapy agents, therefore it is used in the cardio-oncology sector.

In any case, the polyphenol concentrate described herein can also be used for preventing and/or treating damage to the cardiocirculatory system deriving from other conditions, other than treatment with drugs, preferably advanced age, acute or chronic cardiovascular pathologies or harmful lifestyles.

Vegetation waters preferably derive from an olive grinding process with three phases (oil, vegetation waters and pomace) and/or two phases (oil and pomace + vegetation waters).

According to a preferred aspect of the invention, the vegetation waters generated by the oil mill can be treated with a solution with acidic pH that preferably varies between 3 and 5, more preferably the pH is about 4/5. The pH is preferably optimized by adding a strong acid and/or pectolytic enzymes, or enzymes that hydrolyze the cellulosic matrix of olive skin. According to a preferred embodiment of the invention, the pomace is stoned, diluted and/or prefiltered. The pomace preferably has a size that ranges from 0.5 to 1 millimetre (mm), more preferably of about 0.7 mm. An example of the size is that obtained with vibrating screen type sieving. The stoned pomace is possibly dissolved and/or dispersed in an aqueous matrix with pH preferably comprised between 3 and 5, more preferably between 3.5 and 4.

The dissolving step has the aim of dissolving the polyphenols that would otherwise remain trapped in the solid matrix of the olive skins.

In a preferred embodiment of the invention, the concentrate further comprises: at least one further phenol compound preferably selected from: tyrosol, chlorogenic acid, β-hydroxyverbascoide, rutin, verbascoide, and luteolin; and/or at least one metal preferably selected from: sodium, calcium, magnesium and potassium; and/or at least an anion preferably selected from: chlorides, sulphates, phosphates and nitrates; and/or at least one carbohydrate selected from: glucose, fructose, mannitol and sucrose.

In a further embodiment of the invention the concentrate comprises nitrogenous substances (proteins, aminoacids), preferably in a quantity comprised between 15 and 60 mg/kg, more preferably between 20 and 40 mg/kg (mg of nitrogen per litre of active solution).

In any case the phenol compounds contained in the highest quantity in the concentrate are hydroxytyrosol and 3,4-DHPA-EDA.

Preferably, the quantity of hydroxytyrosol ranges between 1 and 10 grams per litre of vegetation waters (g/L), more preferably between 1.5 and 5 g/L, even more preferably between 2 and 3 g/L.

Preferably the quantity of tyrosol is comprised between 0.1 and 0.4 g/L, more preferably between 0.15 g/L and 0.25 g/L.

Preferably the quantity of chlorogenic acid is comprised between 0.06 and 0.24 g/L, more preferably between 0.8 and 0.16 g/L.

Preferably the quantity of β-hydroxyverbascoide is comprised between 0.3 and 1.5, more preferably between 0.5 and 1 g/L.

Preferably the quantity of rutin is comprised between 0.05 and 0.2 g/L, more preferably between 0.08 and 0.15 g/L.

Preferably the quantity of verbascoide is comprised between 0.4 and 1.7 g/L, more preferably between 0.6 and 1 g/L.

Preferably the quantity of luteolin is comprised between 0.1 and 0.5 g/L, more preferably between 0.15 and 0.28 g/L.

Preferably the quantity of sodium is comprised between 75 and 300 mg/L, more preferably between 120 and 180 mg/L.

Preferably the quantity of calcium is comprised between 5 and 10 g/L, more preferably between 2 and 5 g/L.

Preferably the quantity of magnesium is comprised between 220 and 900 mg/L, more preferably between 400 and 500 mg/L.

Preferably the quantity of potassium is comprised between 3 and 15 g/L, more preferably between 6 and 9 g/L.

Preferably the quantity of chlorides is comprised between 1.5 and 7 g/L, more preferably between 2.5 and 4.5 g/L.

Preferably the quantity of sulphates is comprised between 12 and 45 g/L, more preferably between 18 and 28 g/L.

Preferably the quantity of phosphates is comprised between 1.5 and 7 g/L, more preferably between 2.5 and 5 g/L.

Preferably the quantity of nitrates is comprised between 12 and 50 mg/L, more preferably between 18 and 30 mg/L.

Preferably the quantity of glucose is comprised between 15 and 60 g/L, more preferably between 25 and 35 g/L.

Preferably the quantity of fructose is comprised between 3.5 and 15 g/L, more preferably between 5 and 9 g/L.

Preferably the quantity of mannitol is comprised between 1 and 4 g/L, more preferably between 1.5 and 3 g/L.

Preferably the quantity of sucrose is comprised between 4 and 16 g/L, more preferably between 6 and 10 g/L.

In a preferred embodiment of the invention the concentrate is obtained/obtainable through a process comprising the steps of:
(i) microfiltering a sample of the vegetation waters and/or pomace so as to obtain a concentrate and a permeate of microfiltration; and
(ii) concentrating by reverse osmosis the microfiltration permeate obtained in step (i).

According to a preferred aspect of the invention, microfiltration is performed after the dissolving step as described above.

Microfiltration has the purpose of separating a concentrate, i.e. the concentrated fraction of the suspended content of the vegetation waters/pomace, e.g. micro-fragments, fibres and corpuscular material such as cells and bacteria. It is performed under standard conditions for this type of matrix.

As well as the concentrate, following the microfiltration step a permeate is obtained, i.e. a clear fraction, characterized by a colour that varies according to the starting material and that contains the dissolved components of the vegetation waters/pomace, e.g. proteins, sugars, salts, polyphenols, organic acids and various soluble organic molecules.

Preferably the microfiltration is performed with at least one, preferably two, ceramic membranes(s). The membrane is characterized by a preferably tubular shape. In a preferred embodiment the membrane is made of alumina oxide and zirconia.

According to a preferred aspect of the invention, the membrane has the following characteristics:
- an outer diameter that ranges from about 30 to about 40 mm, preferably about 25 mm; and/or
- a length that ranges from about 500 to about 1500 mm, preferably about 1200 mm; and/or
- a series of channels with diameter, preferably hydraulic, that ranges from about 2.5 to about 5 mm, preferably about 3.5 mm; and/or
- a filtering surface area that ranges from about 0.15 to about 0.7 m², preferably about 0.35 m²; and/or
- a molecular size that ranges from about 0.1 micron to about 300 kDa.

The reverse osmosis step, for concentrating the permeate obtained from the microfiltration of the vegetation waters/pomace as described above, is performed under standard conditions for this type of matrix, preferably through the use of a polymeric membrane, more preferably polyamide. Preferably, the membrane has a wound spiral shape and/or a molecular size with high salt rejection, i.e. able to reject the sodium chloride molecule with a percentage of 99.9%. This means that the osmosis membrane retains the molecules of biomedical interest and only lets through the water molecule.

Preferably, the polymeric membrane has a filtering surface area that ranges from about 5 to about 15 m², preferably about 7 m².

The reverse osmosis step allows the permeate obtained from microfiltration to be concentrated preferably about 4 times, which means that from 100 L of microfiltration permeate, 25 L of concentrate are obtained. In this case the volume concentration ratio (VCR) is 4 i.e. 100/25.

The VCR can change based on the starting matrix (vegetation waters) and especially based on its salt content, as the reverse osmosis process must counterbalance the osmotic pressure of the matrix that is to be concentrated.

Further subject matter of the present disclosure is constituted by a concentrate (or phytocomplex) of vegetation waters/pomace obtainable/obtained with the process described above.

Preferably said concentrate comprises the substances specified above in the relative quantities, in particular it is hydroxytyrosol and 3,4-DHPA-EDA, where the quantity of 3,4-DHPA-EDA is preferably comprised between 0.5 and 8 g/L, more preferably between 1 and 6 g/L, even more preferably between 1.5 and 2.5 g/L and/or the quantity of tyrosol is preferably comprised between 0.1 and 0.4 g/L, more preferably between 0.15 g/L and 0.25 g/L. In other words, the concentrate preferably has the composition described above in relation to the content of phenol compounds, and/or metals and/or carbohydrates, and/or anions and/or nitrogen.

A further aspect of the present disclosure relates to a pharmaceutical composition comprising said concentrate and further excipients/pharmacologically accepted ingredients.

A further aspect of the present disclosure relates to a composition comprising the concentrate as described above and further excipients/pharmacologically accepted ingredients and the use thereof in the prevention and/or treatment of damage to the cardiovascular system (tissues/related organs), preferably damage from chemotherapy and/or radiotherapy as described above.

According to a further aspect of the disclosure, said concentrate and/or composition is/are used, alone or in combination with other substances, compounds, drugs or compositions with a protective and/or curative action for damage to cardiovascular tissue, preferably selected from: beta blockers, ACE-inhibitors, anticoagulant statins, dexrazoxane, N-acetyl-I-cysteine(NAC), zinc, selenium, melatonin, L-carnitine, glutathione and coenzyme Q10 (Albini A. et al, JNCI 2009, PMID:20007921).

Therefore, the concentrate can be applied for preventive purposes to prevent lesions/damage and/or for curative purposes for treating lesions/damage affecting the heart tissue and/or the vascular tissue.

In one embodiment, the treatment of the tissue coating the blood vessels is of particular interest, or the endothelial cells and/or the vascular support cells, such as pericytes and/or smooth muscular cells, and/or of the tissue coating the pericardium and/or endocardium muscle. In a particularly preferred embodiment, it is possible to use the concentrate and/or composition described herein for the purpose of preventing and/or treating lesions on the tissue coating the blood vessels and the heart muscle in patients subjected to pharmacological treatment, in particular treatment with antineoplastic agents; in fact it is known that treatment with such drugs for curing cancer cells can weaken the blood vessel walls and the heart muscle itself, with a consequent increase in cardiovascular risk in patients subjected to chemotherapy.

The concentrate of vegetation waters and/or pomace and/or the composition for the uses described in detail above is/are preferably formulated for oral intake, preferably as a beverage. Said beverage as well as the concentrate and/or composition according to the invention comprises one or more possible excipients normally contained in the formulation of various types of beverages. Preferably, the beverage may be based on water and/or fruit and/or milk. In a particularly preferred embodiment of the invention, the beverage is based on fruit, preferably based on grapes. In particular, grape juice and/or must is preferred, preferably of organic grapes.

It is a functional beverage, i.e. to be used as a food supplement because of the therapeutic effects found and described herein.

Alternatively, the concentrate of vegetation waters and/or pomace described herein and/or the composition can be prepared in the form of oral formulations of various kinds, such as sweets, pills, tablets or granules.

Practically the beverage or oral formulation can be taken as a food supplement, in particular for the purpose of preventing and/or treating damage or lesions to the cardiac and or/vascular tissue. In a preferred embodiment, the beverage or oral formulation is taken as a food supplement in order to prevent and/or treat damage or lesions to the cardiac and/or vascular tissue in patients subjected to cardiotoxic pharmacological therapies, more in particular in oncology patients subjected to chemotherapy treatment with antineoplastic drugs.

Possibly said oral formulation can be taken in association with one or more further substances, compounds, drugs or compositions with a protective action against damage to the cardiovascular system.

Alternatively, the concentrate of vegetation waters and/or pomace and/or the composition for the uses described in detail above is/are formulated, preferably for topical application, as a cream, oil, ointment, aerosol, shampoo, detergent, gel, ovule or mouthwash. The concentrate and/or composition can also be in the form of powder, granules, for example following a drying and/or freeze drying process. Such product can be used for preventing and/or treating cardiovascular damage, also topically, in particular in the case of preventing and/or treating the vascular tissue. In a preferred embodiment, the above product can therefore be used to protect or regenerate the vascular tissue with a topical action. Therefore, a further aspect of the present invention relates to a support comprising the concentrate and/or the composition of the present invention. Said support is preferably a patch, bandage, gauze, spray, solution or cardiac device such as, for example, a stent, comprising the concentrate and/or the composition of the present invention. The concentrate and/or composition on said support and/or powder may comprise further agents/molecules characterised by a biologically relevant function for the purposes of the prevention and/or treatment of cardiovascular damage.

### EXAMPLE

### Evaluation of the effect of the polyphenol concentrate on the viability of zebrafish (Danio rerio) embryos and on their heart rate.

To evaluate the cardioprotective effect of the polyphenol concentrate of the invention, the zebrafish was used in vivo as the animal model to study. In particular the experiments were performed on zebrafish (*Danio rerio*) embryos incubated for 24, 48 and 72 hours after fertilization.

For the purpose of inducing cardiotoxicity the drug doxorubicin was used, a commonly used chemotherapy agent, whose cardiotoxic effects are known, and having a similar biochemical/molecular base to the one found for other drugs with cardiotoxic potential, not necessarily of the chemotherapy type.

With reference to Fig.1, it is observed that the treatment of zebrafish embryo with doxorubicin (3 µg/ml) significantly reduces their viability between 24 and 72 hours post-fertilization (hpf). In detail, 10 embryos are used for experimental conditions (compound concentration and unit of time of the treatment). The viability was evaluated by counting the number of dead embryos (per concentration and unit of time). The embryo is considered dead in its "coagulated" state i.e. with a black/brown colour (presence of necrosis).

The results show that concomitant or preventive treatment with the polyphenol concentrate of the invention (at the dilutions 1:500 and 1:1000) significantly reduces the damage induced by doxorubicin. The effect of the polyphenol concentrate is only displayed as a result of cardiac damage as the polyphenol concentrate alone does not alter the viability of the embryos, as can be noted from Fig.1.

Furthermore, it has been observed that the treatment of zebrafish embryos with doxorubicin (3 µg/ml) significantly reduces their heart rate at 48 hours (Fig. 2) and 72 hours (Fig.3). On this point, the results show that concomitant or previous treatment with the polyphenol concentrate of the invention (at the dilutions 1:500 and 1:1000) can reverse the cardiotoxic effect and restore the heart rate. Also in this case, it was noted that the effect of the polyphenol concentrate is only displayed as a result of cardiac damage as the polyphenol concentrate alone does not alter the heart rate of the embryos (Fig.2 and 3).

## Claims

1. Concentrate of olive vegetation waters and/or olive pomace comprising hydroxytyrosol and 3,4- DHPA -EDA and/or a composition comprising said concentrate wherein said concentrate is obtained by a process comprising the steps of:
(i) microfiltering a sample of the vegetation water and/or olive pomace so as to obtain a concentrate and a permeate of microfiltration; and
(ii) concentrating by reverse osmosis the microfiltration permeate of step (i),for use in the prevention and/or in the treatment of damages to the cardiovascular system, preferably to the myocardium, more preferably to the cardiomyocytes, wherein the quantity of 3,4-DHPA-EDA is comprised between 0.5 and 8 g/L and wherein the damages are caused by drug and/or chemotherapy agent having a cardiotoxic effect.

2. The concentrate and/or the composition for use according to claim 1, wherein the quantity of 3,4-DHPA-EDA is comprised between 1 and 6 g/L

3. The concentrate and/or the composition for use according to claim 1, wherein the quantity of 3,4-DHPA-EDA is comprised between 1.5 and 2.5 g/L.

4. The concentrate and/or the composition for use according to anyone of claims 1-3 further comprising:
- at least one phenolic compound preferably selected from: tyrosol, chlorogenic acid, β-hydroxyverbascoside, rutin, verbascoside, and luteolin; and/or
- at least one metal preferably selected from: sodium, calcium, magnesium and potassium; and/or
- at least an anion preferably selected from: chlorides, sulphates, phosphates and nitrates; and/or
- at least one carbohydrate selected from: glucose, fructose, mannitol and sucrose; and/or
- nitrogen.

5. The concentrate and/or the composition for use according to any one of claims 1-4, wherein the microfiltration step involves the use of at least one ceramic membrane, preferably **characterized by** a tubular shape, more preferably made by alumina oxide and zirconia.

6. The concentrate and/or the composition for use according to any one of claims 1-5, wherein the reverse osmosis is performed by using a polimeric membrane, preferably made of polyamide, said membrane being preferably **characterized by** a spiral shape.

7. The concentrate and/or the composition for use according to any one of claims 1-6, wherein said damages are selected from: hypertensive crisis, pulse alterations, arrhythmia, ischemia, preferably caused by vasospasm, and/or thrombo-hemolytic phenomena, fibrosis, senescence, loss of contractile activity, production of reactive oxygen species, autophagy and any combination thereof.

8. The concentrate and/or the composition for use according to any one of claims 1-7 in combination with other substances, compounds, drugs or protective and/or curative compositions for cardiovascular system damages, preferably selected from: beta blockers, ACE inhibitors, anti-coagulants, N-acetyl-L-cysteine (NAC), zinc, selenium, melatonin, L-carnitine / acetyl-L-carnitine, glutathione, coenzyme Q10, flavonoids.

9. The concentrate and/or the composition for use according to any one of claims 1-8, wherein said concentrate and /or said composition is in the form for oral intake, preferably as a beverage wherein said beverage is preferably water- and/or fruit- and/or milk-based, more preferably is based on juice and/or grape must.

10. The concentrate and/or the composition for use according to any one of claims 1-8, wherein said concentrate and/or said composition is formulated, preferably for topical applications preferably a patch, bandage, gauze, spray, solution or cardiac device, preferably a stent.

## Patentansprüche

1. Konzentrat aus Olivenvegetationswasser und/oder Oliventrester, umfassend Hydroxytyrosol und 3,4-DHPA-EDA und/oder eine Zusammensetzung, umfassend das Konzentrat, wobei das Konzentrat durch ein Verfahren erhalten wird, umfassend die Schritte:
(i) Mikrofiltrieren einer Probe des Vegetationswassers und/oder Oliventrester, um ein Konzentrat und ein Permeat der Mikrofiltration zu erhalten; und
(ii) Konzentrieren des Mikrofiltrationspermeats aus Schritt (i) durch Umkehrosmose zur Verwendung bei der Prävention und/oder Behandlung von Schäden des kardiovaskulären Systems, vorzugsweise des Myokards, bevorzugter der Kardiomyozyten, wobei die Menge an 3,4-DHPA-EDA zwischen 0,5 und 8 g/L liegt und wobei die Schäden durch Arzneimittel und/oder Chemotherapeutikum mit kardiotoxischer Wirkung verursacht werden.

2. Konzentrat und/oder Zusammensetzung zur Verwendung nach Anspruch 1, wobei die Menge an 3,4-DHPA-EDA zwischen 1 und 6 g/L liegt.

3. Konzentrat und/oder Zusammensetzung zur Verwendung nach Anspruch 1, wobei die Menge an 3,4-DHPA-EDA zwischen 1,5 und 2,5 g/L liegt.

4. Konzentrat und/oder Zusammensetzung zur Verwendung nach einem der Ansprüche 1-3, ferner umfassend:
- mindestens eine phenolische Verbindung, vorzugsweise ausgewählt aus: Tyrosol, Chlorogensäure, β-Hydroxyverbascosid, Rutin, Verbascosid und Luteolin; und/oder
- mindestens ein Metall, vorzugsweise ausgewählt aus: Natrium, Calcium, Magnesium und Kalium; und/oder
- mindestens ein Anion, vorzugsweise ausgewählt aus: Chloriden, Sulfaten, Phosphaten und Nitraten; und/oder
- mindestens ein Kohlenhydrat ausgewählt aus: Glucose, Fructose, Mannitol und Saccharose; und/oder
- Stickstoff.

5. Konzentrat und/oder Zusammensetzung zur Verwendung nach einem der Ansprüche 1-4, wobei der Mikrofiltrationsschritt die Verwendung mindestens einer keramischen Membran beinhaltet, die vorzugsweise durch eine röhrenförmige Form gekennzeichnet ist, die bevorzugter durch Aluminiumoxid und Zirkonoxid hergestellt ist.

6. Konzentrat und/oder Zusammensetzung zur Verwendung nach einem der Ansprüche 1-5, wobei die Umkehrosmose unter Verwendung einer polimeren Membran, vorzugsweise aus Polyamid, durchgeführt wird, wobei die Membran vorzugsweise durch eine Spiralform gekennzeichnet ist.

7. Konzentrat und/oder Zusammensetzung zur Verwendung nach einem der Ansprüche 1-6, wobei die Schäden ausgewählt sind aus: hypertensiver Krise, Pulsveränderungen, Arrhythmie, Ischämie, vorzugsweise verursacht durch Vasospasmus und/oder thrombohämolytische Phänomene, Fibrose, Seneszenz, Verlust der kontraktilen Aktivität, Produktion reaktiver Sauerstoffspezies, Autophagie und einer beliebigen Kombination davon.

8. Konzentrat und/oder Zusammensetzung zur Verwendung nach einem der Ansprüche 1-7 in Kombination mit anderen Substanzen, Verbindungen, Arzneimitteln oder schützenden und/oder heilenden Zusammensetzungen für Schäden des kardiovaskulären Systems, vorzugsweise ausgewählt aus: Betablockern, ACE-Hemmern, Antikoagulanzien, N-Acetyl-L-Cystein (NAC), Zink, Selen, Melatonin, L-Carnitin / Acetyl-L-Carnitin, Glutathion, Coenzym Q10, Flavonoiden.

9. Konzentrat und/oder Zusammensetzung zur Verwendung nach einem der Ansprüche 1-8, wobei das Konzentrat und/oder die Zusammensetzung in der Form zur oralen Einnahme vorliegt, vorzugsweise als Getränk, wobei das Getränk vorzugsweise auf Wasser- und/oder Frucht- und/oder Milchbasis ist, bevorzugter auf Saft- und/oder Traubenmostbasis.

10. Konzentrat und/oder Zusammensetzung zur Verwendung nach einem der Ansprüche 1-8, wobei das Konzentrat und/oder die Zusammensetzung, vorzugsweise für topische Anwendungen vorzugsweise ein Pflaster, Verband, Gaze, Spray, Lösung oder Herzvorrichtung, vorzugsweise ein Stent, formuliert ist.

## Revendications

1. Concentré d'eau de végétation d'olive et/ou marc d'olive comprenant de l'hydroxytyrosol et du 3,4-DHPA-EDA et/ou une composition comprenant ledit concentré, dans lequel ledit concentré est obtenu par un procédé comprenant les étapes suivantes :
(i) microfiltrer un échantillon d'eau de végétation d'olive et/ou marc d'olive de manière à obtenir un concentré et un perméat de microfiltration ; et
(ii) concentrer par osmose inverse le perméat de microfiltration de l'étape (i), destiné à être utilisé dans la prévention et/ou le traitement de dommages au système cardiovasculaire, de préférence au myocarde, et plus préférablement aux cardiomyocytes, dans lequel la quantité de 3,4-DHPA-EDA est comprise entre 0,5 et 8 g/L et dans lequel les dommages sont causées par un médicament et/ou un agent de chimiothérapie ayant un effet cardiotoxique.

2. Concentré et/ou composition destinés à être utilisés selon la revendication 1, dans lesquels la quantité de 3,4-DHPA-EDA est comprise entre 1 et 6 g/L.

3. Concentré et/ou composition destinés à être utilisés selon la revendication 1, dans lesquels la quantité de 3,4-DHPA-EDA est comprise entre 1,5 et 2,5 g/L.

4. Concentré et/ou composition destinés à être utilisés selon l'une quelconque des revendications 1-3, comprenant en outre :
- au moins un composé phénolique choisi de préférence parmi : le tyrosol, l'acide chlorogénique, le β-hydroxyverbascoside, la rutine, le verbascoside et la lutéoline ; et/ou
- au moins un métal choisi de préférence parmi : le sodium, le calcium, le magnésium et le potassium ; et/ou
- au moins un anion choisi de préférence parmi : les chlorures, les sulfates, les phosphates et les nitrates ; et/ou
- au moins un glucide choisi parmi : le glucose, le fructose, le mannitol et le saccharose ; et/ou
- de l'azote.

5. Concentré et/ou composition destinés à être utilisés selon l'une quelconque des revendications 1-4, dans lesquels l'étape de microfiltration implique l'utilisation d'au moins une membrane céramique, de préférence **caractérisée par** une forme tubulaire, de préférence encore constituée d'oxyde d'alumine et de zircone.

6. Concentré et/ou composition destinés à être utilisés selon l'une quelconque des revendications 1-5, dans lesquels l'osmose inverse est réalisée à l'aide d'une membrane polymère, de préférence en polyamide, ladite membrane étant de préférence **caractérisée par** une forme en spirale.

7. Concentré et/ou composition destinés à être utilisés selon l'une quelconque des revendications 1-6, dans lesquels lesdites dommages sont choisies parmi : une crise d'hypertension, des altérations du pouls, une arythmie, une ischémie, de préférence provoquée par un vasospasme, et/ou des phénomènes thrombotiques et hémolytiques, une fibrose, la sénescence, une perte d'activité contractile, la production d'espèces réactives de l'oxygène, l'autophagie et toute combinaison de ceux-ci.

8. Concentré et/ou composition destinés à être utilisés selon l'une quelconque des revendications 1-7, en combinaison avec d'autres substances, composés, médicaments ou compositions protectrices et/ou curatives contre les dommages au système cardiovasculaire, choisis de préférence parmi : les bêtabloquants, les inhibiteurs de l'ECA, les anticoagulants, la N-acétyl-L-cystéine (NAC), le zinc, le sélénium, la mélatonine, la L-carnitine / acétyl-L-carnitine, le glutathion, la coenzyme Q10, les flavonoïdes.

9. Concentré et/ou composition destinés à être utilisés selon l'une quelconque des revendications 1-8, dans lesquels ledit concentré et/ou ladite composition se présentent sous une forme destinée à l'ingestion par voie orale, de préférence sous forme de boisson, ladite boisson étant de préférence à base d'eau et/ou de fruits et/ou de lait, et de préférence encore à base de jus et/ou de moût de raisin.

10. Concentré et/ou composition destinés à être utilisés selon l'une quelconque des revendications 1-8, dans lesquels ledit concentré et/ou ladite composition sont formulés, de préférence pour des applications topiques, notamment sous forme de patch, de bandage, de gaze, de spray, de solution ou de dispositif cardiaque, de préférence un stent.
